# EUROPÄISCHE PATENTSCHRIFT

(11) **EP 1 845 298 B1**
(45) Veröffentlichungstag und Bekanntmachung des Hinweises auf die Patenterteilung: **11.01.2012**
(21) Anmeldenummer: 07006731.9
(22) Anmeldetag: 30.03.2007
(51) Int. Cl.: F16L 37/098, A61B 17/34, F16L 37/00, F16L 41/02, F16L 47/32

(54) **Kupplung für rohrförmige Elemente**
Coupling for tubular elements
Couplage pour éléments tubulaires

(30) Priorität: 03.04.2006 DE 102006016211
(43) Veröffentlichungstag der Anmeldung: 17.10.2007
(73) Patentinhaber: NovaLung GmbH, 72379 Hechingen (DE)
(72) Erfinder: Spranger, Martin, 72379 Hechingen (DE); Joost, Thilo, 72379 Hechingen (DE)
(74) Vertreter: Graf von Stosch, Andreas

(56) Entgegenhaltungen:
- DE-A1- 19 626 016
- FR-A1- 2 808 071
- US-A- 4 707 000
- US-A- 6 158 467

## Beschreibung

Die vorliegende Erfindung betrifft eine Kupplung für rohrförmige Elemente mit zumindest einem Stecker und einer Buchse, wobei der Stecker in die Buchse einführbar und darin verrastbar ist, und wobei sowohl der Stecker als auch die Buchse in etwa in ihrer Längsrichtung einen Kanal für ein Fluid aufweisen, der so angeordnet ist, dass in einem verrasteten Zustand das Fluid durch die Kupplung hindurch fließen kann.

Die Erfindung betrifft ferner eine Kanüle, die eine solche Kupplung aufweist.

Zur Kupplung rohrförmiger Elemente wurden lange Zeit kurze Rohrabschnitte verwendet, deren Außendurchmesser geringfügig größer war als der Innendurchmesser der zu koppelnden rohrförmigen Elemente. Die zu verbindenden rohrförmigen Elemente wurden dabei einfach auf die Kupplung aufgeschoben, wobei der Unterschied im Durchmesser für die feste Verbindung gesorgt hat. Gegebenenfalls waren noch Riffelungen an der Kupplung angebracht, um für eine noch sicherere Verbindung zu sorgen.

Eine solche Art der Verbindung hat den Nachteil, dass häufig große Kräfte notwendig sind, um die rohrförmigen Elemente auf die Kupplung aufzuschieben, was insbesondere auf dem Gebiet der Medizintechnik häufig unerwünscht ist, da die so ausgeübten Kräfte auf einen Patienten übertragen werden können und dort zu Traumata führen können.

Zur Lösung dieses Problems ist z.B. aus dem US-Patent 5,052,725 eine zweiteilige Kupplung bekannt, die aus einem Stecker und einer Buchse besteht, wobei die zu verbindenden rohrförmigen Elemente jeweils in unverbundenem Zustand mit dem Stecker bzw. der Buchse verbunden werden können und diese dann ohne übermäßigen Kraftaufwand untereinander verbunden werden können.

Es hat sich nun allerdings gezeigt, dass es bei solchen Kupplungen oft zu unerwünschten Luftansammlungen kommen kann. Ferner ist es insbesondere im Bereich der Medizintechnik häufig wünschenswert, wenn in das in den rohrförmigen Elementen transportierte Fluid weitere Substanzen eingeführt werden können oder Proben aus diesem Fluid entnommen werden können.

Ein solches Entnehmen von Proben oder Einführen von weiteren Substanzen ist bei den bekannten Kupplungen nur dadurch möglich, dass die Verbindung gelöst, die gewünschte Tätigkeit durchgeführt und die Verbindung danach wieder hergestellt wird. Das Entfernen von unerwünschter Luft kann ebenfalls nur über ein Trennen und Wiederverbinden der Kupplung erfolgen. Dies ist aufwändig und macht ein häufiges Unterbrechen des Fluidstroms durch die Kupplung notwendig.

Eine weitere gattungsgemäße Kupplung ist aus dem US-Patent 6,158,467 bekannt.

Es ist somit Aufgabe der Erfindung, eine Kupplung für rohrförmige Elemente zu beschreiben, die auf einfache Weise entlüftet werden kann und bei der auch im Betrieb Substanzen von außen in den Fluidstrom eingebracht oder Proben aus dem Fluidstrom entnommen werden können.

Erfindungsgemäß wird die Aufgabe durch die Merkmale des Anspruchs 1 gelöst.

Unter einem rohrförmigen Element gemäß der Erfindung wird ein langgestrecktes Gebilde verstanden, das in etwa entlang seiner Längsachse zumindest einen Kanal zum Transport eines Fluids aufweist. Zu solchen rohrförmigen Elementen gehören z.B. sowohl starre Röhren als auch flexible Schläuche.

Das Verrasten erfolgt durch Nuten in Kombination mit Rastnasen, so dass eine feste, aber lösbare Verbindung geschaffen wird.

Durch die Bereitstellung der Öffnung ist es nun möglich, den Stecker und die Buchse der Kupplung miteinander zu kuppeln, wobei in einer ersten Raststellung noch ein Zugang von außen zu dem innenliegenden Kanal für ein Fluid besteht, so dass über diesen die Kupplung entlüftet werden kann oder aus dem Fluidstrom Substanzen entnommen oder diesen zugeführt werden können. In einer zweiten Raststellung wird die Öffnung verschlossen, und der Kanal ist vollständig von der Umgebung abgeschnitten.

Auf diese Weise ist z.B. ein Entlüften der Kupplung möglich, ohne dass dabei die Kupplung gelöst werden müsste, und ohne dass dabei der Fluidstrom durch die Kupplung unterbrochen werden muss. Da während dieser Zeit der Stecker mit der Buchse verbunden bleibt, kann der Zugang zum Fluidstrom außerdem schneller erfolgen. Dadurch dass der Stecker und die Buchse in Verbindung bleiben, ist außerdem das Öffnen und Schließen der Öffnung deutlich einfacher und kann im Einhandbetrieb erfolgen.

Es ist hierbei verständlich, dass die Ausdrücke "erste Raststellung" und "zweite Raststellung" lediglich zur Abgrenzung der Raststellungen untereinander dienen und keine Aussage über deren Abfolge machen. Es ist durchaus denkbar, dass beim Einführen des Steckers in die Buchse die zweite Raststellung vor der ersten Raststellung erreicht wird.

In einer Ausgestaltung der Erfindung ist ein Betätigungselement vorhanden, mit dem das Verrasten des Steckers mit der Buchse gelöst werden kann.

Auch wenn es möglich ist, eine Verrastung zu bauen, die alleine durch Zug an Stecker und Buchse gelöst werden kann, wird bei einer genügend sicheren Verbindung zum Lösen einer solchen Verbindung eine relativ große Kraft benötigt, die z.B. auf einen Patienten übertragen werden kann und dort zu Traumata führt. Durch das Vorsehen eines Betätigungselements kann die Verrastung gelöst werden, ohne dass auf die Kupplung übermäßige Kräfte ausgeübt werden können. Der Stecker und die Buchse können somit von der einen in die andere Raststellung überführt oder voneinander getrennt werden, ohne dass besondere Kraft nötig ist, so dass dies auch im Einhandbetrieb möglich ist. Die Art des Betätigungselements hängt hierbei von der Art des Rastmechanismus ab, und dieses kann z.B. einen Druckknopf aufweisen.

Gemäß der Erfindung weist der Stecker an einer äußeren Mantelfläche zumindest zwei Ringnuten auf, und die Buchse weist zumindest eine Rastnase auf, die in Raststellung jeweils in eine Ringnut eingreift.

Eine solche Konstruktion hat sich sowohl in der Herstellung als auch in der Bedienung als besonders einfach erwiesen und führt gleichzeitig zu einer ausreichend sicheren Verrastung zwischen dem Stecker und der Buchse. Das Vorsehen von Ringnuten ermöglicht ferner das Verdrehen von Stecker und Buchse gegeneinander im verrasteten Zustand.

In einer Ausgestaltung der oben genannten Maßnahme wird die Rastnase durch einen geschlitzten Ring gebildet, der in einer Ringnut in einer inneren Mantelfläche der Buchse angeordnet ist und zumindest an einer Stelle über die innere Mantelfläche herausragt, wobei insbesondere das Betätigungselement einen Keil aufweist, mit dem der geschlitzte Ring so spreizbar ist, dass ein Eingreifen des Rings in eine der Ringnuten des Steckers gelöst werden kann.

Gemäß dieser Ausführungsform können die Rastnasen z.B. durch zwei Seiten eines Rings gebildet werden, die über die innere Mantelfläche der Buchse hinausstehen und in den Raststellungen in die Ringnuten des Steckers eingreifen. Wird nun ein Keil in den Schlitz des Rings eingeführt, werden die beiden überstehenden Seiten des Rings nach außen gedrückt und greifen nicht mehr in die Nuten ein. Somit wird die Verrastung gelöst.

Auf diese Weise kann die Buchse bis auf den Ring einstückig hergestellt werden, wobei der Ring z.B. auf einem normalen geschlitzten Federring basieren kann, was diese Konstruktion einfach und preiswert in der Herstellung macht. Außerdem kann durch Verwendung eines Rings die Verrastung über einen relativ großen Teil der Mantelfläche des Steckers erfolgen, was die Verbindung besonders sicher macht.

Gemäß der Erfindung weist die Öffnung an der Außenseite einen Anschluss auf, der eine Buchse für eine Spritze ist.

Dieses Merkmal hat sich besonders bei der Anwendung der Kupplung auf dem Gebiet der Medizintechnik als vorteilhaft erwiesen, da damit auf einfache Weise mit einer Spritze Proben aus dem Fluidstrom entnommen bzw. Substanzen in den Fluidstrom eingebracht werden können. Ferner kann über einen solchen Anschluss auch kontrolliert entlüftet werden, woraus sich z.B. Hinweise ergeben können, ob es in dem System Leckagen gibt.

In einer weiteren Ausgestaltung der Erfindung bestehen der Stecker und die Buchse aus Kunststoff.

Hierdurch wird eine Kupplung geschaffen, die sowohl einfach und preiswert in der Herstellung als auch leicht ist. Ferner können dadurch Stecker und Kupplung als Einmalartikel ausgelegt sein, was insbesondere auf dem medizinischen Sektor vorteilhaft ist, da dadurch die Kupplung nicht mehr resterilisiert werden muss.

In einer weiteren Ausgestaltung der Erfindung weist der Stecker im vorderen Bereich eine Dichtung auf.

Durch diese Maßnahme wird eine besonders dichte Verbindung sichergestellt.

In einer weiteren Ausführungsform ist die Öffnung in der Buchse angeordnet.

Durch diese Maßnahme kann auch in der Raststellung, in der der Kanal von der Umgebung isoliert ist, die Öffnung z.B. mit einer Spritze verbunden werden, so dass eine Probe entnommen werden kann, ohne dass es zu einem übermäßigen Flüssigkeitsaustritt kommt.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird nun anhand der beiliegenden Zeichnungen näher erläutert. Es zeigen:
- Fig. 1: einen Stecker und eine Buchse im Schnitt im nichtverrasteten Zustand,
- Fig. 2: einen Stecker und eine Buchse im Schnitt in einer ersten Raststellung,
- Fig. 3: einen Stecker und eine Buchse im Schnitt in einer zweiten Raststellung, und
- Fig. 4: eine perspektivische Ansicht einer Kanüle mit einer Kupplung.

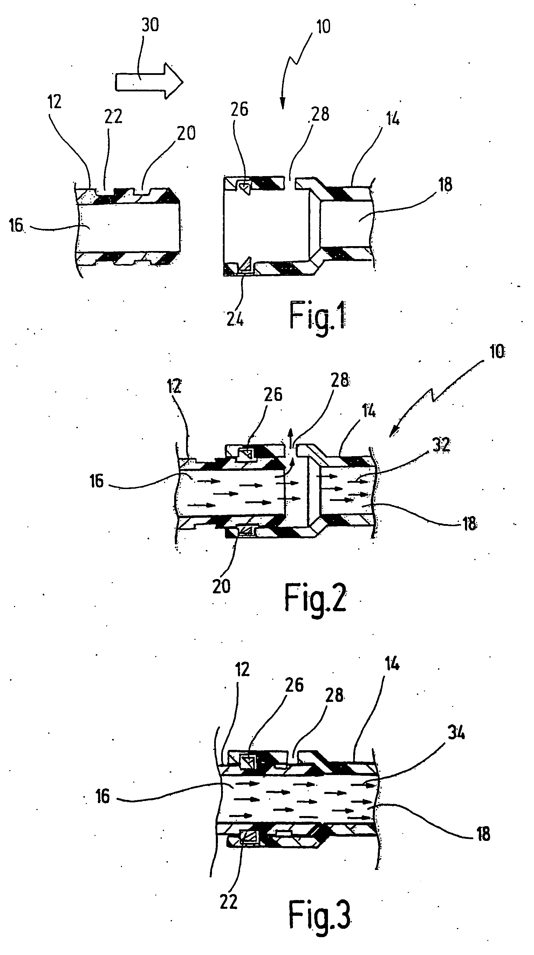

In Fig. 1 ist eine Kupplung in ihrer Gesamtheit mit der Bezugsziffer 10 bezeichnet.

Die Kupplung 10 weist einen Stecker 12 und eine Buchse 14 auf. Der Stecker 12 weist entlang seiner Längsachse einen Kanal 16 für ein Fluid auf, wobei die Buchse 14 entlang ihrer Längsachse einen entsprechenden Kanal 18 aufweist. Sowohl der Stecker 12 als auch die Buchse 14 bestehen hier aus Kunststoff.

In einer äußeren Mantelfläche des Steckers 12 sind eine erste Ringnut 20 und eine zweite Ringnut 22 eingebracht.

In einer inneren Mantelfläche der Buchse 14 ist eine Ringnut 24 eingebracht. In dieser Ringnut 24 ist ein geschlitzter Ring 26 angeordnet, der einen dreieckigen Querschnitt aufweist. Der Ring 26 ist so in der Ringnut 24 angeordnet, dass zumindest ein Teil davon über die innere Mantelfläche der Buchse 14 hinausragt.

Die Buchse 14 weist ferner eine Öffnung 28 auf, die hier als einfache Bohrung ausgeführt ist, durch die der Kanal 18 mit der Umgebung verbunden ist.

Die Kupplung des Steckers 12 mit der Buchse 14 erfolgt nun durch Einführen des Steckers 12 in die Buchse 14 in Richtung eines Pfeils 30.

In Fig. 2 ist die Kupplung 10 in einer ersten Raststellung nach der in Fig. 1 durch den Pfeil 30 angedeuteten Bewegung dargestellt. In dieser Darstellung wird sichtbar, dass der Ring 26 nun in die erste Ringnut 20 des Steckers 12 eingreift. Somit sind der Stecker 12 und die Buchse 14 fest verrastet miteinander verbunden.

Der Kanal 18 ist hierbei noch immer über die Öffnung 28 mit der Umgebung verbunden. Fließt nun ein Fluid, wie dieses durch die Pfeile 32 angedeutet ist, in diesem Fall Blut eines Patienten, das in einen extrakorporalen Kreislauf abgeführt wird durch den Kanal 16 bzw. 18, so kann dieses durch die Öffnung 28 in die Umgebung austreten. Hat sich nun an der Kupplung 10 Luft angesammelt, wird diese durch den Fluidstrom 32 durch die Öffnung 28 nach außen herausgedrückt, und die Kupplung 10 wird somit entlüftet.

In Fig. 3 ist die Situation nach einer weiteren Bewegung, wie sie in Fig. 1 durch den Pfeil 30 angedeutet ist, dargestellt. Hierbei wurde der Stecker 12 weiter in die Buchse 14 eingeführt. Der Ring 26 kommt nun in der zweiten Ringnut 22 zu liegen und verrastet den Stecker 12 in dieser Stellung in der Buchse 14.

Ferner wird aus dieser Darstellung deutlich, dass nun die Öffnung 28 durch den Stecker 12 blockiert ist, so dass der Kanal, der durch die Kanäle 16 und 18 gebildet wird, vollkommen von der Umgebung isoliert ist und ein Strom des Fluids nur noch durch die Kanäle 16 und 18 möglich ist, wie dieses durch die Pfeile 34 angezeigt ist.

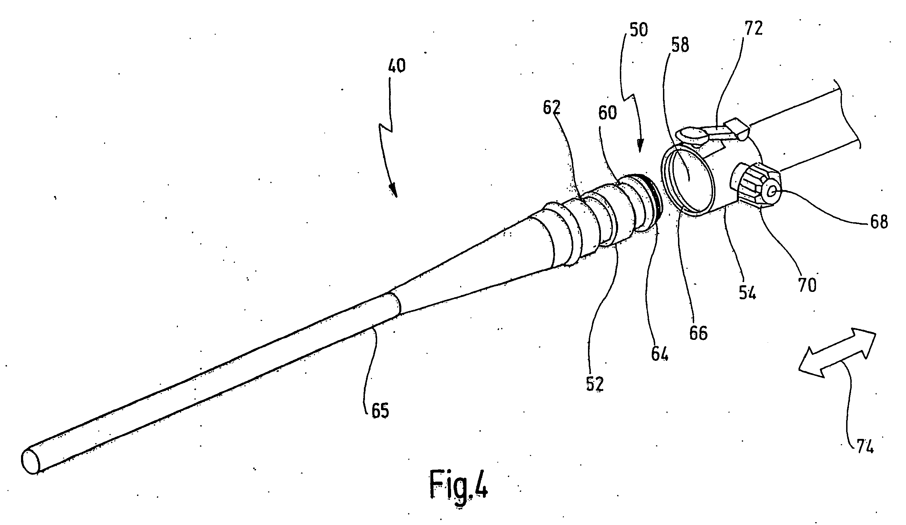

In Fig. 4 ist eine Kanüle in ihrer Gesamtheit mit der Bezugsziffer 40 bezeichnet.

Diese Kanüle 40 weist eine Kupplung 50 auf, die aus einem Stecker 52 und einer Buchse 54 besteht. In der Buchse 54 ist ein Kanal 58, der zur Durchleitung eines Fluids dient, sichtbar.

In einer äußeren Mantelfläche des Steckers 52 sind zwei Ringnuten 60 und 62 eingebracht, wobei im vorderen Bereich des Steckers 52 ferner eine Dichtung 64 angeordnet ist. Auf der der Dichtung 64 gegenüberliegenden Seite schließt sich an den Stecker die eigentliche Kanüle 65 an, die hier als übergangslose Kanüle ausgebildet ist. Diese Kanüle 65 besteht aus einem drahtverstärkten Schlauch, der auf einen Konus aus Kunststoff aufgesetzt ist, wobei die Drahtverstärkung zwischen einer inneren und einer äußeren Schicht des Schlauches angeordnet ist.

In der inneren Mantelfläche der Buchse 54 ist ein geschlitzter Ring 66 angeordnet, der dazu dient, den Stecker 52 in der Buchse 54 zu verrasten. An der Seite der Buchse 54 ist eine Öffnung 68 angeordnet, die hier einen Anschluss 70 in Form einer Buchse für eine Spritze aufweist. Durch diese Öffnung 68 ist der Kanal 58 der Buchse 54 mit der Umgebung verbunden.

Die Buchse 54 weist ferner ein Betätigungselement 72 auf, das hier als Druckknopf ausgebildet ist. Mit diesem Druckknopf kann ein Keil in einen in dem Ring 66 vorhandenen Schlitz hineingedrückt werden, wodurch der Ring 66 gespreizt wird. Durch die Spreizung des Rings 66 kann die Verrastung des Steckers 52 in der Buchse 54 gelöst werden, und die beiden Teile können voneinander getrennt werden.

Während der Verwendung z.B. als Kanüle zum Ableiten von Blut in einen extrakorporalen Blutkreislauf wird nun die Kanüle 65 mit Hilfe eines Dilatators in ein Gefäß eingebracht. Der Dilatator kann dabei z.B. über die Ringnut 60 fest mit der Kanüle 65 verbunden sein.

Nach dem Setzen der Kanüle wird gegebenenfalls der Dilatator entfernt, und die Buchse 54, die an ihrem von der Kanüle 65 entfernten Ende mit einem Schlauch verbunden ist, wird in Richtung eines Doppelpfeils 74 nach links auf den Stecker 52 aufgesetzt. An dem Anschluss 70 ist hierbei eine hier nicht dargestellte Spritze angeordnet. Die Buchse 54 wird nun so weit in Richtung des Doppelpfeils 74 nach links bewegt, bis der Ring 66 in der ersten Ringnut 60 des Steckers 52 zu liegen kommt. In diesem Zustand ist der Kanal 58 der Buchse 54 noch über die Öffnung 68 mit der Umgebung, in diesem Fall mit der Spritze, verbunden.

Ein Operateur kann nun durch ein Aufziehen der Spritze prüfen, ob an der Kupplungsstelle Luftblasen vorhanden sind, bzw. er kann mittels der Spritze über den Anschluss 70 eine Blutprobe entnehmen. Wenn sich der Operateur vom ordnungsgemäßen Zustand der Kupplung überzeugt hat, bewegt er die Buchse 54 weiter in Richtung des Doppelpfeils 74 nach links, wodurch der Ring 66 jetzt in der zweiten Ringnut 62 zu liegen kommt und der Stecker 52 die Öffnung 68 innen verschließt, so dass der innere Kanal nun vollständig von der Umgebung isoliert ist.

Wünscht ein Operateur nun z.B. ein Medikament in den Blutkreislauf einzuspritzen, kann er dieses in eine Spritze aufnehmen und diese Spritze in den Anschluss 70 einsetzen. Hiernach betätigt er das Betätigungselement 72, wodurch die Verrastung des Steckers 52 in der Buchse 54 gelöst wird. Er bewegt nun die Buchse 54 in Richtung des Doppelpfeils 74 nach rechts, und zwar so weit, dass der Ring 66 jetzt wieder in der ersten Ringnut 60 des Steckers 52 zu liegen kommt. Dadurch ist der Kanal 58 wiederum über die Öffnung 68 mit der Umgebung verbunden, und über die Spritze kann das gewünschte Medikament in den Blutstrom injiziert werden.

Nach Abschluss des Einspritzens bewegt der Operateur die Buchse 54 wiederum in Richtung des Doppelpfeils 74 nach links, wodurch die Öffnung 68 durch den Stecker 52 verschlossen wird.

Nach Ende der Prozedur oder wenn die Kanüle mit einem anderen Schlauch verbunden werden soll, betätigt ein Operateur wiederum das Betätigungselement 72, wodurch der Ring 66 gespreizt wird und nicht mehr in die Ringnut 62 des Steckers 52 eingreift. Der Operateur bewegt nun die Buchse 54 in Richtung des Doppelpfeils 74 nach rechts und trennt diese vollständig von dem Stecker 52, ohne dass dabei besondere Kräfte auf den Patienten ausgeübt werden.

Der Stecker 52 kann nun entweder mit einem anderen schlauchförmigen Element gekoppelt, oder die Kanüle 65 kann aus dem Patienten entfernt werden.

## Patentansprüche

1. Kupplung für rohrförmige Elemente mit zumindest einem Stecker (12; 52) und einer Buchse (14; 54), wobei der Stecker (12; 52) in die Buchse (14; 54) einführbar und darin verrastbar ist, und wobei sowohl der Stecker (12; 52) als auch die Buchse (14; 54) in etwa entlang ihrer Längsachse einen Kanal (16, 18; 58) für ein Fluid aufweisen, der so angeordnet ist, dass in einem verrasteten Zustand das Fluid durch die Kupplung (10; 50) hindurch fließen kann, wobei der Stecker (12; 52) in zumindest zwei Raststellungen in der Buchse (14; 54) verrastbar ist und die Buchse (14; 54) und/oder der Stecker (12; 52) zumindest eine Öffnung (28; 68) aufweist, so dass in einer ersten Raststellung die Öffnung (28; 68) den Kanal (16, 18; 58) für ein Fluid mit der Umgebung verbindet, und in einer zweiten Raststellung der Kanal (16, 18; 58) für ein Fluid von der Umgebung isoliert ist, wobei die Öffnung (28; 68) an der Außenseite einen Anschluss (70) aufweist, wobei der Anschluss (70) eine Buchse für eine Spritze ist, **dadurch gekennzeichnet, dass** der Stecker (12; 52) an einer äußeren Mantelfläche zumindest zwei Ringnuten (20, 22; 60, 62) aufweist und dass die Buchse (14; 54) zumindest eine Rastnase aufweist, die in den Rastellungen jeweils in eine der Ringnuten (20, 22; 60, 62) eingreift,

2. Kupplung nach Anspruch 1, **dadurch gekennzeichnet, dass** ein Betätigungselement (72) vorhanden ist, mit dem das Verrasten des Steckers (12; 52) mit der Buchse (14; 54) gelöst werden kann.

3. Kupplung nach Anspruch 1 oder 2, **dadurch gekennzeichnet, dass** die Rastnase durch einen geschlitzten Ring (26; 66) gebildet wird, der in einer Ringnut (24) in einer inneren Mantelfläche der Buchse (14; 54) angeordnet ist und zumindest an einer Stelle über die innere Mantelfläche herausragt.

4. Kupplung nach Anspruch 3, **dadurch gekennzeichnet, dass** das Betätigungselement einen Keil (72) aufweist, mit dem der geschlitzte Ring so spreizbar ist, dass ein Eingreifen des Rings in eine der Ringnuten (20, 22; 60, 62) des Steckers (12; 52) gelöst werden kann.

5. Kupplung nach einem der Ansprüche 1 bis 4, **dadurch gekennzeichnet, dass** der Stecker (12; 52) und die Buchse (14; 54) aus Kunststoff bestehen.

6. Kupplung nach einem der Ansprüche 1 bis 5, **dadurch gekennzeichnet, dass** der Stecker (12; 52) im vorderen Bereich eine Dichtung (64) aufweist.

7. Kupplung nach einem der Ansprüche 1 bis 6, **dadurch gekennzeichnet, dass** die Öffnung (28; 68) in der Buchse (14; 54) angeordnet ist.

8. Kanüle, die eine Kupplung (10; 50) nach einem der Ansprüche 1 bis 7 aufweist.

## Claims

1. Coupling for tubular elements, with at least one plug (12; 52) and one socket (14; 54), wherein the plug (12; 52) can be inserted into the socket (14; 54) and can be locked therein, and wherein both the plug (12; 52) and the socket (14; 54) have, extending approximately along their longitudinal axis, a channel (16, 18; 58) for a fluid, said channel being arranged in such a way that the fluid can flow through the coupling (10; 50) in a locked state thereof, wherein the plug (12; 52) can be locked in the socket (14; 54) in at least two locking positions, and wherein the socket (14; 54) and/or the plug (12; 52) comprises at least one opening (28; 68) such that, in a first locking position, the opening (28; 68) connects the channel (16, 18; 58) for a fluid to the environment, and, in a second locking position, the channel (16, 18; 58) for a fluid is isolated from the environment, wherein the opening (28; 68) on the outer face has a connector piece (70) wherein the connector piece (70) is a socket for a syringe, **characterized in that** the plug (12; 52) has at least two annular grooves (20, 22; 60, 62) on an outer circumferential surface, and **in that** the socket (14; 54) has at least one locking lug that engages in one of the annular grooves (20, 22; 60, 62) in the locking positions.

2. Coupling according to claim 1, **characterized in that** an actuating element (72) is present with which the locked engagement of the plug (12; 52) to the socket (14; 54) can be cancelled.

3. Coupling according to claim 1 or claim 2, **characterized in that** the locking lug is formed by a slotted ring (26; 66) which is arranged in an annular groove (24) in an inner circumferential surface of the socket (14; 54) and protrudes above the inner circumferential surface at least at one location.

4. Coupling according to claim 3, **characterized in that** the actuating element comprises a wedge (72) with which the slotted ring can be spread open in such a way that an engagement of the ring in one of the annular grooves (20, 22; 60, 62) of the plug (12; 52) can be cancelled.

5. Coupling according to any one of claims 1 to 4, **characterized in that** the plug (12; 52) and the socket (14; 54) are made of plastic.

6. Coupling according to any one of claims 1 to 5, **characterized in that** the plug (12; 52) has a seal (64) in the front area.

7. Coupling according to any one of claims 1 to 6, **characterized in that** the opening (28; 68) is arranged in the socket (14; 54).

8. Cannula, comprising a coupling (10; 50) according to any one of claims 1 to 7.

## Revendications

1. Accouplement pour éléments tubulaires, comprenant au moins un connecteur mâle (12; 52) et un connecteur femelle (14 ; 54), le connecteur mâle (12; 52) pouvant être introduit dans le connecteur femelle (14 ; 54) et y être encliqueté, et tant le connecteur mâle (12; 52) que le connecteur femelle (14; 54) comportent approximativement le long de leur axe longitudinal un canal (16, 18 ; 58) pour un fluide, qui est disposé de telle sorte que, dans un état encliqueté, le fluide peut s'écouler à travers l'accouplement (10 ; 50), le connecteur mâle (12 ; 52) pouvant être encliqueté dans au moins deux positions d'encliquetage dans le connecteur femelle (14 ; 54) et le connecteur femelle (14 ; 54) et/ou le connecteur mâle (12 ; 52) comportant au moins une ouverture (28 ; 68), de sorte que, dans une première position d'encliquetage, l'ouverture (28 ; 68) relie le canal (16, 18 ; 58) pour un fluide à son milieu extérieur et que, dans une deuxième position d'encliquetage, le canal (16, 18 ; 58) pour un fluide est isolé de son milieu extérieur, l'ouverture (28 ; 68) comportant sur le côté extérieur un raccord (70), le raccord (70) étant un connecteur femelle pour une seringue, **caractérisé en ce que** le connecteur mâle (12 ; 52) comporte sur une surface circonférentielle extérieure au moins deux rainures annulaires (20, 22 ; 60, 62), et **en ce que** le connecteur femelle (14 ; 54) comporte au moins un bec d'encliquetage qui, dans les positions d'encliquetage, s'engage respectivement dans une des rainures annulaires (20, 22 ; 60, 62).

2. Accouplement selon la revendication 1, **caractérisé en ce qu'**un élément d'actionnement (72) est présent, qui permet de défaire l'assemblage par encliquetage du connecteur mâle (12; 52) avec le connecteur femelle (14; 54).

3. Accouplement selon la revendication 1 ou 2, **caractérisé en ce que** le bec d'encliquetage est formé par un anneau fendu (26 ; 66), qui est disposé dans une rainure annulaire (24) dans une surface circonférentielle intérieure du connecteur femelle (14 ; 54) et dépasse de la surface circonférentielle intérieure au moins en un endroit.

4. Accouplement selon la revendication 3, **caractérisé en ce que** l'élément d'actionnement comporte une clavette (72) au moyen de laquelle l'anneau fendu peut être écarté de telle sorte qu'un engagement de l'anneau dans une des rainures annulaires (20, 22 ; 60, 62) du connecteur mâle (12; 52) peut être défait.

5. Accouplement selon l'une quelconque des revendications 1 à 4, **caractérisé en ce que** le connecteur mâle (12 ; 52) et le connecteur femelle (14 ; 54) sont réalisés en matière plastique.

6. Accouplement selon l'une quelconque des revendications 1 à 5, **caractérisé en ce que** le connecteur mâle (12 ; 52) comporte un joint d'étanchéité (64) dans la région avant.

7. Accouplement selon l'une quelconque des revendications 1 à 6, **caractérisé en ce que** l'ouverture (28 ; 68) est disposée dans le connecteur femelle (14 ; 54).

8. Canule, qui comporte un accouplement (10 ; 50) selon l'une quelconque des revendications 1 à 7.
